# EUROPEAN PATENT APPLICATION

(11) **EP 4 843 619 A1**
(43) Date of publication of application: **25.11.2026**
(21) Application number: 26180108.8
(22) Date of filing: 21.05.2026
(51) Int. Cl.: A61N 5/10

(54) **TREATMENT-PLAN GENERATION METHOD, TREATMENT- PLAN EXECUTION METHOD, ELECTRONIC DEVICE, AND RADIOTHERAPY SYSTEM**

(30) Priority: 23.05.2025 CN 202510676848
(71) Applicant: Our United Corporation, Xi'an, Shaanxi 710018 (CN)
(72) Inventor: YANG, Weifeng, Shaanxi, 710018 (CN)
(74) Representative: Murgitroyd & Company

(57) **Abstract**

A treatment-plan generation method includes: obtaining a target image of a target object, contour information of a target volume, and a prescription requirement; defining a treatment field of the target volume, and marking a treatment-field type for the treatment field, wherein the treatment-field type indicates a treatment mode corresponding to the treatment field, and wherein the treatment mode comprises at least one of a treatment mode distinguished by radiation type or a treatment mode distinguished by irradiation type; and generating a fused treatment plan based on the target image, the contour information of the target volume, the treatment field, the treatment-field type, and the prescription requirement, wherein the fused treatment plan comprises treatment-plan parameters for treatment fields of different treatment-field types.

## Description

### CROSS-REFERENCE TO RELATED APPLICATION

This application claims priority to Chinese Patent Application No. 202510676848.4, filed on May 23, 2025, the entire contents of which are incorporated herein by reference.

### BACKGROUND OF THE INVENTION

### Field of the Invention

The present application relates to radiotherapy technologies, and more particularly to a treatment-plan generation method, a treatment-plan execution method, an electronic device, and a radiotherapy system.

### Description of the Related Art

Radiotherapy is an effective and important modality for treating tumors. In tumor treatment, two common types of radiotherapy devices are Gamma Knife systems and accelerators.

Gamma Knife systems are widely used in cranial neurosurgery and employ multi-source stereotactic focusing technology. Gamma Knife systems may provide a high dose rate at a focal region, concentrated dose distribution, a small error range, and a short treatment cycle, and therefore may be suitable for treating relatively small tumors. Accelerators are widely used for treating body tumors and employ rotational intensity-modulated conformal irradiation. Accelerators may be advantageous for radiotherapy of relatively large tumors, for example by inhibiting tumor growth and improving a patient's post-treatment quality of life.

Because accelerators and Gamma Knife systems are suitable for different clinical scenarios, a physician typically selects which radiotherapy device is to be used for treatment. However, clinical scenarios may be more complex. In some cases, advantages of a Gamma Knife system and an accelerator may be combined, and two treatment approaches may be used together, for example, by treating a tumor through conventional radiotherapy and a local boost.

During multi-modality combined treatment of a tumor, different radiotherapy devices may generate treatment plans separately, for example, a Gamma Knife plan and an accelerator plan may be generated separately, followed by separate scheduling and treatment. This may increase the time required for treatment planning and delivery and may reduce efficiency.

### SUMMARY OF THE INVENTION

A treatment-plan generation method, a treatment-plan execution method, and an electronic device are provided. The treatment-plan generation method improves treatment-plan generation efficiency and accuracy.

According to a first aspect, a treatment-plan generation method is provided. The method includes: obtaining, by a treatment planning device, a target image of a target object, contour information of a target volume, and a prescription requirement; defining, by the treatment planning device, a treatment field of the target volume, and marking a treatment-field type for the treatment field, wherein the treatment-field type indicates a treatment mode corresponding to the treatment field, and wherein the treatment mode includes at least one of a treatment mode distinguished by radiation type or a treatment mode distinguished by irradiation type; and generating, by the treatment planning device, a fused treatment plan based on the target image, the contour information of the target volume, the treatment field, the treatment-field type, and the prescription requirement, wherein the fused treatment plan includes treatment-plan parameters for treatment fields of different treatment-field types.

In some examples, the treatment-plan generation method further includes: grouping, by the treatment planning device, treatment fields in the target volume based on treatment-field types of the treatment fields, to obtain a plurality of groups of treatment fields; determining, by the treatment planning device, a dose field of each group of treatment fields among the plurality of groups of treatment fields and superposing dose fields of the plurality of groups of treatment fields to obtain a dose field of the target volume; and optimizing, by the treatment planning device, the fused treatment plan based on the dose field of the target volume.

In some examples, superposing the dose fields of the plurality of groups of treatment fields to obtain the dose field of the target volume includes: partitioning, by the treatment planning device according to a preset lattice size, the dose field of each group of treatment fields among the plurality of groups of treatment fields to obtain a dose lattice of each group of treatment fields; and superposing, by the treatment planning device, dose lattice elements located at corresponding positions in the dose lattices of the plurality of groups of treatment fields to obtain the dose field of the target volume.

In some examples, the prescription requirement includes prescription requirements for a plurality of target volumes of different types in the target volume.

In some examples, the prescription requirement is obtained by: obtaining, by the treatment planning device in response to a marking operation for a target-volume type of the target volume, the target-volume type of the target volume; and setting, by the treatment planning device based on the target-volume type of the target volume, prescription requirements respectively for target volumes of different target-volume types.

According to a second aspect, a treatment-plan execution method is provided. The method includes: determining, by a radiotherapy device, a current treatment fraction; obtaining, by the radiotherapy device from a fused treatment plan, treatment-plan parameters of treatment fields associated with the current treatment fraction, wherein the fused treatment plan is generated based on the treatment-plan generation method according to the first aspect, the fused treatment plan includes treatment-plan parameters for treatment fields of different treatment-field types, and the treatment fields associated with the current treatment fraction include treatment fields of at least one treatment-field type; and executing, by the radiotherapy device, a treatment plan of the current treatment fraction based on the treatment-plan parameters of the treatment fields associated with the current treatment fraction.

In some examples, the treatment-plan parameters include a treatment-head irradiation angle for the treatment field. When a plurality of treatment fields are associated with the current treatment fraction, executing, by the radiotherapy device, the treatment plan of the current treatment fraction based on the treatment-plan parameters of the treatment fields associated with the current treatment fraction includes: obtaining, by the radiotherapy device from the treatment-plan parameters of the treatment fields associated with the current treatment fraction, a treatment-head irradiation angle for each treatment field associated with the current treatment fraction; ordering, by the radiotherapy device according to a preset order, the treatment-head irradiation angles for the treatment fields associated with the current treatment fraction to obtain an ordering result; and executing, by the radiotherapy device based on the treatment-plan parameters of the treatment fields associated with the current treatment fraction and the ordering result, the treatment plan of the current treatment fraction.

In some examples, the treatment-plan execution method further includes: determining, by the radiotherapy device, a treatment field at which execution of the treatment plan of the current treatment fraction is interrupted, when execution of the treatment plan of the current treatment fraction is interrupted; and continuing, by the radiotherapy device in response to an instruction for resuming execution of the treatment plan of the current treatment fraction, execution of the treatment plan of the current treatment fraction based on treatment-plan parameters for the treatment field at which execution of the current treatment fraction is interrupted.

According to a third aspect, an electronic device is provided. The electronic device includes a processor and a memory configured to store instructions executable by the processor. The processor is configured to execute the instructions to implement the treatment-plan generation method according to the first aspect or the treatment-plan execution method according to the second aspect.

According to a fourth aspect, a non-transitory computer-readable storage medium storing computer instructions is provided. The computer instructions, when read and executed, implement the treatment-plan generation method according to the first aspect or the treatment-plan execution method according to the second aspect.

According to a fifth aspect, a radiotherapy system includes a treatment planning device and a radiotherapy device. The treatment planning device is configured to implement the treatment-plan generation method according to the first aspect, and the radiotherapy device is configured to implement the treatment-plan execution method according to the second aspect.

The radiotherapy device includes one of a multi-head radiotherapy host, a single-head multi-mode radiotherapy host, or a radiotherapy host system including a plurality of radiotherapy hosts. The multi-head radiotherapy host includes a plurality of treatment heads, and the plurality of treatment heads are configured to emit at least one of beams of different radiation types or beams of different irradiation types. The single-head multi-mode radiotherapy host includes one treatment head configured to emit beams of a plurality of different irradiation types. The radiotherapy host system including the plurality of radiotherapy hosts includes a plurality of different radiotherapy hosts, the different radiotherapy hosts correspond to different treatment modes, and the treatment modes include at least one of treatment modes distinguished by radiation type or treatment modes distinguished by irradiation type.

Accordingly, the present application may define and mark different types of treatment fields for a target volume, and a generated fused treatment plan may include treatment-plan parameters for treatment fields of different treatment-field types. Therefore, the present application may generate a fused treatment plan for at least one of a treatment mode distinguished by radiation type or a treatment mode distinguished by irradiation type, thereby improving treatment-plan generation efficiency and accuracy and reducing cumbersome scheduling and treatment processes and low efficiency caused by generating different treatment plans for different treatment modes.

### BRIEF DESCRIPTION OF THE DRAWINGS

The accompanying drawings facilitate understanding of embodiments described herein and are not intended to limit the scope of the claims.
FIG. 1 is a schematic structural diagram of a radiotherapy system according to an embodiment of the present application.
FIG. 2 is a schematic flowchart of a treatment-plan generation method according to an embodiment of the present application.
FIG. 3 is a schematic diagram of a target volume according to an embodiment of the present application.
FIG. 4 is a schematic diagram illustrating content of a fused treatment plan based on a DICOM standard format according to an embodiment of the present application.
FIG. 5 is another schematic flowchart of a treatment-plan generation method according to an embodiment of the present application.
FIG. 6 is a schematic diagram of another treatment-plan generation method according to an embodiment of the present application.
FIG. 7 is a schematic flowchart of a treatment-plan execution method according to an embodiment of the present application.
FIG. 8 is another schematic flowchart of a treatment-plan execution method according to an embodiment of the present application.
FIG. 9 is another schematic flowchart of a treatment-plan execution method according to an embodiment of the present application.
FIG. 10 is a schematic structural diagram of a treatment-plan generation apparatus according to an embodiment of the present application.
FIG. 11 is a schematic structural diagram of a treatment-plan execution apparatus according to an embodiment of the present application.
FIG. 12 is a schematic block diagram of an electronic device according to an embodiment of the present application.

### DETAILED DESCRIPTION

Embodiments of the present application are described below with reference to the accompanying drawings. The described embodiments are illustrative and are not intended to limit the present application. Other embodiments and modifications based on the disclosure herein are within the scope of the claims.

In the description of the present application, it should be understood that terms such as "center" "longitudinal" "transverse" "length" "width" "thickness" "upper" "lower" "front" "rear" "left" "right" "vertical" "horizontal" "top" "bottom" "inside" and "outside" indicate orientations or positional relationships based on the drawings. These terms are used for convenience of description and simplification, and do not indicate or imply that a referenced device or element has a specific orientation or is constructed or operated in a specific orientation. Therefore, these terms should not be construed as limiting the present application. In addition, terms such as "first" "second" and "third" are used for descriptive purposes, and should not be construed as indicating or implying relative importance or implicitly indicating a number of indicated technical features. Thus, a feature defined by "first" "second" or "third" may explicitly or implicitly include one or more such features. In the description of the present application, "a plurality of" means two or more, unless otherwise expressly and specifically defined.

In the description of the present application, the term "exemplary" means serving as an example, instance, or illustration. An embodiment described as "exemplary" in the present application should not be construed as necessarily preferred or advantageous over other embodiments. The following description is provided to enable those skilled in the art to make and use the present application. Details are set forth below for purposes of explanation. It should be understood that those skilled in the art may recognize that the present application may be practiced without relying on these specific details. In other instances, well-known structures and processes are not described in detail to avoid unnecessarily obscuring the description of the present application. Therefore, the present application is not intended to be limited to the illustrated embodiments, but is to be accorded a scope consistent with the principles and features disclosed herein.

Because the treatment-plan generation method provided in some embodiments of the present application is performed by a treatment planning device, objects processed by the treatment planning device are in the form of data or information. For example, time is represented as time information. When dimensions, quantities, positions, or the like are described in the following embodiments, the dimensions, quantities, positions, or the like may be represented by corresponding data to facilitate processing by the treatment planning device.

Radiotherapy is an effective and important modality for treating tumors. In tumor treatment, two common types of radiotherapy devices are Gamma Knife systems and accelerators.

Gamma Knife systems are widely used in cranial neurosurgery and employ multi-source stereotactic focusing technology. Gamma Knife systems may provide a high dose rate at a focal region, concentrated dose distribution, a small error range, and a short treatment cycle, and therefore may be suitable for treating relatively small tumors. Accelerators are widely used for treating body tumors and employ rotational intensity-modulated conformal irradiation. Accelerators may be advantageous for radiotherapy of relatively large tumors, for example by inhibiting tumor growth and improving a patient's post-treatment quality of life.

Because accelerators and Gamma Knife systems are suitable for different clinical scenarios, a physician typically selects which radiotherapy device is to be used for treatment. However, clinical scenarios may be more complex. In some cases, advantages of a Gamma Knife system and an accelerator may be combined, and two treatment approaches may be used together, for example, by treating a tumor through conventional radiotherapy and a local boost.

During multi-modality combined treatment of a tumor, different radiotherapy devices may generate treatment plans separately, for example, a Gamma Knife plan and an accelerator plan may be generated separately, followed by separate scheduling and treatment. This may increase the time required for treatment planning and delivery and may reduce efficiency.

The present application provides a treatment-plan generation method, a treatment-plan execution method, an electronic device, and a radiotherapy system, to improve treatment-plan generation efficiency and accuracy.

According to a first aspect, a treatment-plan generation method includes: obtaining, by a treatment planning device, a target image of a target object, contour information of a target volume, and a prescription requirement; defining, by the treatment planning device, a treatment field of the target volume and marking a treatment-field type for the treatment field, wherein the treatment-field type indicates a treatment mode corresponding to the treatment field, and the treatment mode includes at least one of a treatment mode distinguished by radiation type or a treatment mode distinguished by irradiation type; and generating, by the treatment planning device, a fused treatment plan based on the target image, the contour information of the target volume, the treatment field, the treatment-field type, and the prescription requirement, wherein the fused treatment plan includes treatment-plan parameters for treatment fields of different treatment-field types.

In some examples, the treatment-plan generation method further includes: grouping, by the treatment planning device, treatment fields in the target volume based on treatment-field types of the treatment fields, to obtain a plurality of groups of treatment fields; determining, by the treatment planning device, a dose field of each group of treatment fields among the plurality of groups of treatment fields and superposing dose fields of the plurality of groups of treatment fields to obtain a dose field of the target volume; and optimizing, by the treatment planning device, the fused treatment plan based on the dose field of the target volume.

In some examples, superposing the dose fields of the plurality of groups of treatment fields to obtain the dose field of the target volume includes: partitioning, by the treatment planning device according to a preset lattice size, the dose field of each group of treatment fields among the plurality of groups of treatment fields to obtain a dose lattice of each group of treatment fields; and superposing, by the treatment planning device, dose lattice elements located at corresponding positions in the dose lattices of the plurality of groups of treatment fields to obtain the dose field of the target volume.

In some examples, the prescription requirement includes prescription requirements for a plurality of target volumes of different types in the target volume.

In some examples, the prescription requirement is obtained by: obtaining, by the treatment planning device in response to a marking operation for a target-volume type of the target volume, the target-volume type of the target volume; and setting, by the treatment planning device based on the target-volume type of the target volume, prescription requirements respectively for target volumes of different target-volume types.

According to a second aspect, a treatment-plan execution method includes: determining, by a radiotherapy device, a current treatment fraction; obtaining, by the radiotherapy device from a fused treatment plan, treatment-plan parameters of treatment fields associated with the current treatment fraction, wherein the fused treatment plan is generated based on the treatment-plan generation method according to the first aspect, the fused treatment plan includes treatment-plan parameters for treatment fields of different treatment-field types, and the treatment fields associated with the current treatment fraction include treatment fields of at least one treatment-field type; and executing, by the radiotherapy device, a treatment plan of the current treatment fraction based on the treatment-plan parameters of the treatment fields associated with the current treatment fraction.

In some examples, the treatment-plan parameters include a treatment-head irradiation angle for the treatment field. When a plurality of treatment fields are associated with the current treatment fraction, executing, by the radiotherapy device, the treatment plan of the current treatment fraction based on the treatment-plan parameters of the treatment fields associated with the current treatment fraction includes: obtaining, by the radiotherapy device from the treatment-plan parameters of the treatment fields associated with the current treatment fraction, a treatment-head irradiation angle for each treatment field associated with the current treatment fraction; ordering, by the radiotherapy device according to a preset order, the treatment-head irradiation angles for the treatment fields associated with the current treatment fraction to obtain an ordering result; and executing, by the radiotherapy device based on the treatment-plan parameters of the treatment fields associated with the current treatment fraction and the ordering result, the treatment plan of the current treatment fraction.

In some examples, the treatment-plan execution method further includes: determining, by the radiotherapy device, a treatment field at which execution of the treatment plan of the current treatment fraction is interrupted, when execution of the treatment plan of the current treatment fraction is interrupted; and continuing, by the radiotherapy device in response to an instruction for resuming execution of the treatment plan of the current treatment fraction, execution of the treatment plan of the current treatment fraction based on treatment-plan parameters for the treatment field at which execution of the current treatment fraction is interrupted.

According to a third aspect, an electronic device includes a processor and a memory configured to store instructions executable by the processor. The processor is configured to execute the instructions to implement the treatment-plan generation method according to the first aspect or the treatment-plan execution method according to the second aspect.

According to a fourth aspect, a non-transitory computer-readable storage medium storing computer instructions is provided. The computer instructions, when read and executed, implement the treatment-plan generation method according to the first aspect or the treatment-plan execution method according to the second aspect.

According to a fifth aspect, a radiotherapy system includes a treatment planning device and a radiotherapy device. The treatment planning device is configured to implement the treatment-plan generation method according to the first aspect, and the radiotherapy device is configured to implement the treatment-plan execution method according to the second aspect.

The radiotherapy device includes one of a multi-head radiotherapy host, a single-head multi-mode radiotherapy host, or a radiotherapy host system including a plurality of radiotherapy hosts. The multi-head radiotherapy host includes a plurality of treatment heads, and the plurality of treatment heads are configured to emit at least one of beams of different radiation types or beams of different irradiation types. The single-head multi-mode radiotherapy host includes one treatment head configured to emit beams of a plurality of different irradiation types. The radiotherapy host system including the plurality of radiotherapy hosts includes a plurality of different radiotherapy hosts, the different radiotherapy hosts correspond to different treatment modes, and the treatment modes include at least one of treatment modes distinguished by radiation type or treatment modes distinguished by irradiation type.

Accordingly, the present application may define and mark different types of treatment fields for a target volume, and a generated fused treatment plan may include treatment-plan parameters for treatment fields of different treatment-field types. Therefore, the present application may generate a fused treatment plan for at least one of a treatment mode distinguished by radiation type or a treatment mode distinguished by irradiation type, thereby improving treatment-plan generation efficiency and accuracy and reducing cumbersome scheduling and treatment processes and low efficiency caused by generating different treatment plans for different treatment modes.

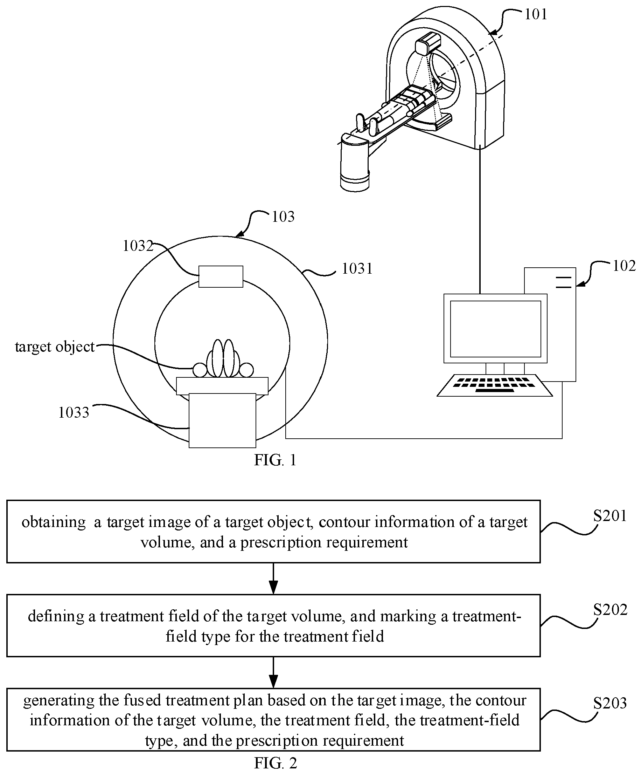

In some embodiments, the treatment-plan generation method and the treatment-plan execution method described herein may be applied to a radiotherapy system. FIG. 1 is a schematic structural diagram of a radiotherapy system according to an embodiment of the present application. Referring to FIG. 1, the radiotherapy system includes an image acquisition device 101, a treatment planning device 102, and a radiotherapy device 103.

The image acquisition device 101 is configured to acquire an image of a tumor site of a target object, such as a target volume or a target volume to be treated, and images of surrounding normal tissues. In some embodiments, the target object may be a patient to be treated, an experimental subject, a phantom, or another suitable treatment object. In some embodiments, the image acquisition device 101 may be at least one of a computed tomography (CT) device, an emission computed tomography (ECT) device, a magnetic resonance imaging (MRI) device, a positron emission tomography (PET) device, or an ultrasound examination device.

The treatment planning device 102 is configured to obtain an image of the target object acquired by the image acquisition device 101 and to generate, optimize, and evaluate a treatment plan. In some embodiments, a treatment planning system (TPS) may run on the treatment planning device 102. The TPS provides functions for treatment-plan generation, optimization, and evaluation. For example, the TPS may be an RT pro TPS system.

In some embodiments, the treatment planning device 102 may include a TPS client and a TPS server.

The TPS client may be at least one of a smart phone, a smart watch, a desktop computer, a laptop computer, a virtual reality terminal, an augmented reality terminal, a wireless terminal, or a portable laptop computer. In some embodiments, a user may run the treatment planning system on the TPS server through the TPS client, thereby causing the TPS server to perform treatment-plan generation and optimization processes. The TPS client may display the generated treatment plan or the optimized treatment plan. In this manner, the time required of the user may be reduced, and the generated or optimized treatment plan may be presented more intuitively for user evaluation.

The TPS server may be at least one of a standalone physical server, a server cluster including multiple physical servers, a distributed file system, or a cloud server that provides basic cloud-computing services, such as cloud services, cloud databases, cloud computing, cloud functions, cloud storage, network services, cloud communications, middleware services, domain-name services, security services, content-delivery networks, big-data platforms, or artificial-intelligence platforms. Some embodiments of the present application are not limited in this respect. The number of TPS servers may vary. The TPS server may also include other functions to provide more comprehensive and diverse services. In some embodiments, the TPS server is configured to provide backend services for the TPS client, such as performing a treatment-plan optimization process in response to a corresponding request from the TPS client.

The radiotherapy device 103 is configured to deliver radiotherapy to the target object. In some embodiments, the radiotherapy device 103 may include a gantry 1031, a treatment head 1032, and a support device 1033. The gantry 1031 may be a rotatable gantry. The treatment head 1032 may be disposed on the gantry 1031 and configured to emit a radiation beam, such as a gamma-ray beam, a megavoltage X-ray beam, or a proton beam, to deliver radiotherapy to the target object. The support device 1033 is configured to support and move the target object. The support device 1033 may be a treatment couch.

In some embodiments, when the target object is on the support device 1033, rotation of the gantry 1031 may drive the treatment head 1032 to irradiate the target object through a 360-degree rotational range, thereby completing radiotherapy.

In some embodiments, the radiotherapy device 103 may obtain and execute a treatment plan generated by the treatment planning device 102.

In some embodiments, different types of radiotherapy devices or treatment modes may be used in the radiotherapy system. For example, a Gamma Knife system may be used in cranial neurosurgery and may employ multi-source stereotactic focusing technology. The Gamma Knife system may provide characteristics such as a high dose rate at a focal region, concentrated dose distribution, a relatively small error range, and a relatively short treatment cycle, and may be suitable for treating relatively small tumors.

In some embodiments, an accelerator may be used to treat body tumors and may employ rotational intensity-modulated conformal irradiation. The accelerator may be suitable for radiotherapy of relatively large tumors, for example by inhibiting tumor growth and improving post-treatment quality of life.

In some clinical scenarios, advantages of a Gamma Knife system and an accelerator may be combined, such that two treatment approaches are used together. For example, a tumor may be treated through conventional radiotherapy and a local boost.

In some implementations, when treatment plans are separately generated for different treatment modes, a physician may use different radiotherapy treatment planning systems to generate corresponding radiotherapy treatment plans and may ultimately superpose radiation doses generated by the different radiotherapy treatment planning systems. In addition, a patient may visit two different treatment rooms at different times for multiple setup and treatment sessions. This may increase patient discomfort and inconvenience for both patients and medical personnel.

Some radiotherapy manufacturers have produced dual-head radiotherapy devices that integrate a Gamma Knife treatment head and an accelerator treatment head into a single device. However, treatment plans for the two treatment approaches are usually still generated separately, followed by separate scheduling and treatment. Such a process may not sufficiently utilize the advantages of the dual-head radiotherapy device and causes unnecessary discomfort and inconvenience for patients and medical personnel.

In some embodiments, the treatment-plan generation method and the treatment-plan execution method may be implemented in a radiotherapy system. The radiotherapy system may include a treatment planning device and a radiotherapy device. The treatment planning device may be configured to implement the treatment-plan generation method described above, and the radiotherapy device may be configured to implement the treatment-plan execution method described above.

In some examples, the radiotherapy device 103 may be one of a multi-head radiotherapy host, a single-head multi-mode radiotherapy host, or a radiotherapy host system including a plurality of radiotherapy hosts.

The multi-head radiotherapy host includes a plurality of treatment heads, and the plurality of treatment heads are configured to emit at least one of beams of different radiation types or beams of different irradiation types.

For example, the multi-head radiotherapy host may include two treatment heads. One treatment head may be a Gamma Knife treatment head for rotational focusing radiotherapy, and the other treatment head may be an accelerator treatment head for intensity-modulated conformal radiotherapy.

The single-head multi-mode radiotherapy host includes one treatment head, and the one treatment head is configured to emit beams of a plurality of different irradiation types.

For example, the treatment head in the single-head multi-mode radiotherapy host may emit the same type of radiation but with different irradiation types, such as a focused irradiation type and an intensity-modulated conformal irradiation type.

The radiotherapy host system including the plurality of radiotherapy hosts includes a plurality of different radiotherapy hosts. The different radiotherapy hosts correspond to different treatment modes, and the treatment modes include at least one of treatment modes distinguished by radiation type or treatment modes distinguished by irradiation type.

For example, the plurality of different radiotherapy hosts may include two hosts. One host may be a radiotherapy host that operates in a rotational focusing radiotherapy mode, and the other host may be a radiotherapy host that operates in an intensity-modulated conformal radiotherapy mode.

Based on the radiotherapy system shown in FIG. 1, the treatment-plan generation method and the treatment-plan execution method provided in some embodiments of the present application are described below.

FIG. 2 is a schematic flowchart of a treatment-plan generation method according to an embodiment of the present application. The treatment-plan generation method may be applied to the treatment planning device 102 shown in FIG. 1. As shown in FIG. 2, the treatment-plan generation method includes the following steps.

S201, obtaining a target image of a target object, contour information of a target volume, and a prescription requirement.

For example, the target object may be a phantom, a human subject, an experimental subject, an animal, or another suitable treatment object. The target volume may be a tumor region of the target object or another region to be treated.

In some embodiments, the image acquisition device may acquire the target image of the target object, and the treatment planning device may obtain the target image acquired by the image acquisition device. In some other embodiments, the treatment planning device may obtain the target image of the target object in response to a medical-record import operation by a user, such as a physician.

The target image of the target object may include an image of the target volume. A contouring operation may be performed on the target image to obtain the contour information of the target volume.

For example, when the treatment planning device obtains the target image acquired by the image acquisition device, the treatment planning device may perform target-volume contouring on the target image to obtain the contour information of the target volume. The contour information may include a contour of a tumor region corresponding to the target volume, contours of organs at risk surrounding the target volume, a skin contour, and the like.

In some examples, the treatment planning device may be connected to a third-party software program. The third-party software program may be used to perform target-volume contouring on the target image to obtain the contour information of the target volume. The third-party software program may also be deployed in the treatment planning device.

In some examples, when target-volume contouring is performed on the target image, a physician may perform a contouring operation on the treatment planning device, and the treatment planning device may obtain the contour information of the target volume in response to the contouring operation performed by the physician.

In some other examples, the treatment planning device may perform automated target-volume contouring on the target image through contouring software to obtain the contour information of the target volume. The contouring software may be installed on a contouring device in a contouring system connected to the treatment planning device. After the contouring device performs target-volume contouring on the target image through the contouring software and obtains the contour information of the target volume, the contouring device may send the contour information of the target volume to the treatment planning device. In some embodiments, the contouring software may also be deployed in the treatment planning device.

The prescription requirement is a specific radiation-dose requirement determined by a physician based on the tumor type, anatomical location, and individual condition of the target object. The prescription requirement may help ensure that the target volume receives a sufficient dose for tumor control while protecting surrounding normal tissues to the extent possible. The prescription requirement is a core basis for treatment-plan generation. In some embodiments, the prescription requirement includes a total dose to be received by the target volume, namely a prescription dose, a number of treatment fractions, namely a number of times the target volume receives treatment, a fraction dose, and dose limits for organs at risk, namely a maximum dose or an average dose that organs at risk surrounding the target volume may receive.

Because the present application may generate a fused treatment plan for treating the target volume under different treatment modes, the prescription requirement in the present application may include prescription requirements for different treatment modes.

In some embodiments, different prescription requirements may be set for different treatment modes. For example, different prescription requirements may be set for different regions based on definitions of regions for different treatment modes in the target image.

In some embodiments, setting different prescription requirements for different treatment modes may include the following steps.

S2011, in response to a marking operation for a target-volume type of the target volume, obtaining the target-volume type of the target volume.

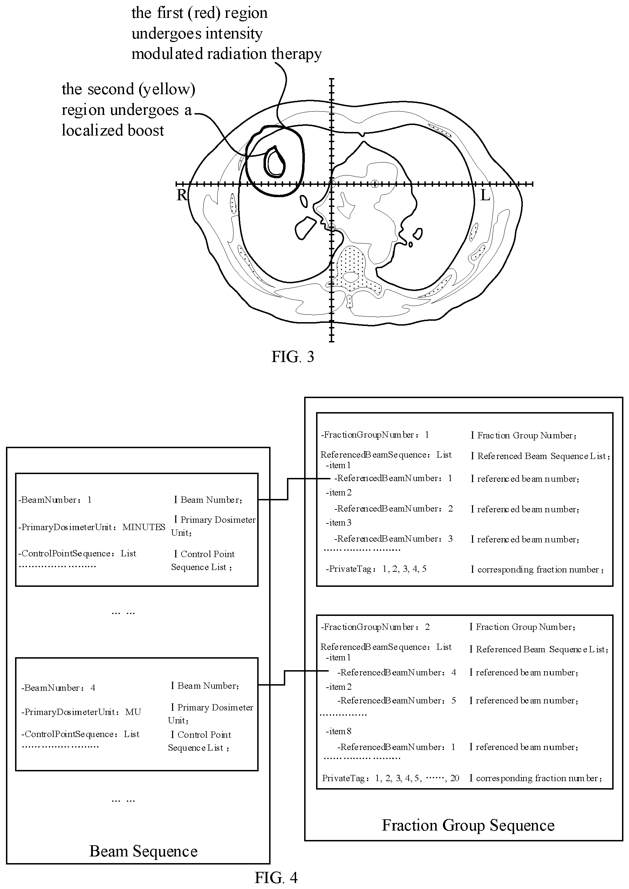

For example, as shown in FIG. 3, after the target image is obtained, target-volume contouring may be performed on the target image to obtain a target volume corresponding to a first (red) region and a target volume corresponding to a second (yellow) region. The target volume corresponding to the first (red) region may receive intensity-modulated radiation therapy over a relatively large region, namely radiotherapy in a linear accelerator treatment mode. In this case, the target volume corresponding to the first (red) region may be marked as an intensity-modulated radiation therapy target volume, namely PTV.

The target volume corresponding to the second (yellow) region may be treated with a local boost by precise supplemental irradiation, namely radiotherapy in a Gamma Knife treatment mode. In this case, the target volume corresponding to the second (yellow) region may be marked as a stereotactic radiotherapy target volume, namely PTV-Gamma. In a black-and-white drawing, the first (red) region and the second (yellow) region shown in FIG. 3 may be represented using different hatch patterns, grayscale levels, or boundary styles.

S2012, based on the target-volume type of the target volume, setting prescription requirements respectively for target volumes of different target-volume types.

Based on the operation of S2011, two target volumes of different types may be obtained, namely the intensity-modulated radiation therapy target volume PTV and the stereotactic radiotherapy target volume PTV-Gamma. Accordingly, prescription requirements may be set respectively for the two target volumes of different types.

For example, in combination with FIG. 3, as shown in Table 1 below, the prescription requirements for the target volumes shown in FIG. 3 may include a prescription requirement for the PTV and a prescription requirement for the PTV-Gamma.

| **Target-volume name** | **Prescription dose (cGy)** | **Prescription isodose line (%)** | **Fraction dose (cGy)** | **Number of treatment fractions** |
|---|---|---|---|---|
| PTV | 3500.0 | 50.0 | 350 | 10 |
| PTV-Gamma | 1200.0 | 95.0 | 400 | 3 |

In some embodiments, the treatment planning device may set an overall prescription requirement for different treatment modes. For example, the treatment planning device may obtain an overall prescription requirement input by a physician and generate a treatment plan based on the overall prescription requirement.

S202, defining a treatment field of the target volume, and marking a treatment-field type for the treatment field.

A treatment field, also referred to as a Beam, refers to an irradiation region formed on a surface of a patient's body or inside the patient by a radiation beam emitted by the radiotherapy device. The treatment field may also be referred to as an irradiation field. When the radiotherapy device is used to treat the target volume, a combination of a plurality of treatment fields may be used to complete treatment of the target volume.

The treatment-field type indicates a treatment mode corresponding to the treatment field. The treatment mode includes at least one of a treatment mode distinguished by radiation type or a treatment mode distinguished by irradiation type.

A treatment mode distinguished by radiation type refers to a treatment mode that is distinguished according to the type of radiation used. The different types of radiation may include, for example, X-rays, gamma rays, proton beams, heavy-ion beams, neutron beams, and the like.

A treatment mode distinguished by irradiation type refers to a treatment mode that is distinguished according to the irradiation approach used. The different irradiation approaches may include, for example, focused treatment, conformal treatment, intensity-modulated treatment, intensity-modulated conformal treatment, and the like.

For example, for a relatively small target volume, a treatment mode corresponding to a treatment field in the target volume may be a Gamma Knife treatment mode. The Gamma Knife treatment mode employs multi-source gamma-ray stereotactic focusing technology, has a high dose rate at a focal region, and may provide concentrated dose distribution, a small error range, and a short treatment cycle. In this case, the treatment field in the target volume may be referred to as a target point, a Gamma Knife irradiation field, a Gamma treatment field, a Gamma field, or another suitable term.

As another example, for a relatively large target volume, a treatment mode corresponding to a treatment field in the target volume may be a linear accelerator treatment mode. The linear accelerator treatment mode is widely used to treat body tumors and employs X-ray rotational intensity-modulated conformal irradiation. The linear accelerator treatment mode may be suitable for radiotherapy of a large target volume, for example, by inhibiting tumor growth and improving the patient's quality of life after treatment. In this case, the treatment field in the target volume may be referred to as a field, an accelerator field, or another suitable term.

It should be understood that, when the target volume includes a plurality of target volumes of different types, different treatment fields may be defined for the target volumes of different types, and treatment-field types may be marked for the treatment fields.

For example, when the target volume includes an intensity-modulated radiation therapy target volume and a stereotactic radiotherapy target volume, the corresponding treatment-field types may include an intensity-modulated treatment field and a focusing treatment field.

Defining the treatment field of the target volume may include determining treatment-plan parameters associated with the treatment field. For example, when the treatment field is a Gamma Knife irradiation field, a target point position, a target point size, a target point weight, or another suitable parameter may be defined. When the treatment field is an accelerator field, an irradiation angle, a field shape, an irradiation duration, or another suitable parameter may be defined.

After the treatment field and the treatment-field type are determined, the treatment planning device generates the fused treatment plan based on the target image, the contour information of the target volume, the treatment field, the treatment-field type, and the prescription requirement. The fused treatment plan includes treatment-plan parameters for treatment fields of different treatment-field types.

In some embodiments, the fused treatment plan may be described using a Digital Imaging and Communications in Medicine (DICOM) standard format. In a conventional process, a Gamma Knife treatment plan and an accelerator treatment plan may each be described using a DICOM standard format. In the present application, parameter fields related to treatment fields in a Gamma Knife treatment plan and parameter fields related to treatment fields in an accelerator treatment plan may be integrated, so that different types of treatment fields may be described in a same fused treatment plan.

Because parameter fields of treatment fields of different treatment-field types may be different, the treatment planning device may obtain a union of parameter fields of the treatment fields of different treatment-field types, so that parameters of treatment fields of different treatment-field types may be included in one treatment plan, namely the fused treatment plan.

For example, when the treatment fields include a Gamma field and an accelerator field, the fused treatment plan may include parameter fields for the Gamma field and parameter fields for the accelerator field.

When the fused treatment plan includes treatment fields of different treatment-field types, the treatment planning device may distinguish or mark the treatment fields of different treatment-field types through a preset field in the DICOM standard format, so that the treatment fields of different treatment-field types may be accurately distinguished and the radiotherapy device may subsequently determine an appropriate treatment mode.

For example, when the treatment fields include a Gamma field and an accelerator field, because the Gamma field and the accelerator field have different dose units, the fused treatment plan may distinguish or mark different types of treatment fields based on a dose-unit field. For the Gamma field, a value of the dose-unit field may be MINUTES. For the accelerator field, a value of the dose-unit field may be MU. Accordingly, the treatment planning device may distinguish or mark the Gamma field and the accelerator field based on the dose-unit field. Subsequently, when the radiotherapy device executes the fused treatment plan, if the radiotherapy device reads that the value of the dose-unit field is MINUTES, the radiotherapy device uses a treatment mode corresponding to the Gamma field for treatment. If the radiotherapy device reads that the value of the dose-unit field is MU, the radiotherapy device uses the treatment mode corresponding to the accelerator field for treatment.

As another example, when the treatment fields include a Gamma field and an accelerator field, because the Gamma field and the accelerator field may have different energy attributes, the fused treatment plan may distinguish or mark different types of treatment fields based on an energy-attribute field. For example, a value of the energy attribute of the Gamma field may be 1.25, and a value of the energy attribute of the accelerator field may be a specific value from 6 to 20. Thus, the Gamma field and the accelerator field may be distinguished or marked based on the value of the energy-attribute field. Subsequently, when the radiotherapy device executes the fused treatment plan, if the radiotherapy device reads that the value of the energy attribute is 1.25, the radiotherapy device uses a treatment mode corresponding to the Gamma field for treatment. If the radiotherapy device reads that the value of the energy attribute is a value from 6 to 20, the radiotherapy device uses the treatment mode corresponding to the accelerator field for treatment.

S203, generating the fused treatment plan based on the target image, the contour information of the target volume, the treatment field, the treatment-field type, and the prescription requirement.

The fused treatment plan includes treatment-plan parameters for treatment fields of different treatment-field types. After obtaining the target image, the contour information of the target volume, the treatment field, the treatment-field type, and the prescription requirement, the treatment planning device may generate the fused treatment plan based on this information.

Accordingly, the present application may define and mark different types of treatment fields for the target volume, and the generated fused treatment plan includes treatment-plan parameters for treatment fields of different treatment-field types. Therefore, the present application may generate a fused treatment plan for at least one of a treatment mode distinguished by radiation type or a treatment mode distinguished by irradiation type, thereby improving treatment-plan generation efficiency and accuracy and reducing cumbersome scheduling and treatment processes and low efficiency caused by generating different treatment plans for different treatment modes.

To ensure that the target volume may receive a total dose specified in the prescription requirement, after the fused treatment plan is generated, treatment fields associated with each treatment fraction may be defined according to the number of treatment fractions set in the prescription requirement.

In some embodiments, in a DICOM standard-format file of the fused treatment plan, treatment fields associated with each treatment fraction may be described through a Fraction Group Sequence field (FractionGroupSequence). The FractionGroupSequence field is a sequence-type field that describes information of treatment fields associated with each treatment fraction in the fused treatment plan.

The FractionGroupSequence field includes a plurality of fraction groups (FractionGroup), and each fraction group corresponds to a specific treatment-field sequence. The fraction group organizes and manages various parameters associated with fractionated irradiation during radiotherapy. Each fraction group may include a Fraction Group Number (FractionGroupNumber).

For example, when the fused treatment plan generated in the present application includes Gamma-field treatment-plan parameters and accelerator-field treatment-plan parameters, the number of fraction groups included in the FractionGroupSequence field may be defined according to the maximum value of the numbers of treatment fractions in the prescription requirements for the two types of treatment fields.

For example, when the number of treatment fractions in the prescription requirement for the Gamma field is 5 and the number of treatment fractions in the prescription requirement for the accelerator field is 20, the number of fraction groups in the FractionGroupSequence field may be defined as 20, so that the FractionGroupSequence field includes 20 fraction groups.

Each fraction group may include a Referenced Beam Sequence field (ReferencedBeamSequence). The ReferencedBeamSequence field describes treatment fields associated with the fraction group. A Referenced Beam Number field (ReferencedBeamNumber) of the treatment field may be used for corresponding marking.

In some examples, to further optimize and combine treatment fraction groups, the 20 fraction groups may be divided into two fraction groups. A first fraction group executes both Gamma-field treatment fields and accelerator-field treatment fields for a total of five fractions respectively associated with fraction numbers 1 to 5. Because five fractions of the Gamma-field treatment fields have already been allocated to the first fraction group, a second fraction group includes accelerator-field treatment fields for a total of 15 fractions respectively associated with fraction numbers 6 to 20. Through this division, the number of fraction groups may be reduced through optimization and combination.

In some examples, the five fractions in which Gamma-field irradiation and accelerator-field irradiation are simultaneously performed may be executed continuously or at intervals. The present application may further define a string-type private tag (Private Tag) to record corresponding fraction numbers in a format such as "1,2,3,4,5", "1-3-5-7-9", or "1/6/11/16/21", thereby indicating specific execution fractions of the fraction group.

For example, "1,2,3,4,5" may indicate that Gamma-field irradiation and accelerator-field irradiation are simultaneously performed for each of the first five fractions. "1-3-5-7-9" may indicate that Gamma-field irradiation and accelerator-field irradiation are simultaneously performed at alternating treatment fractions. "1/6/11/16/21" may indicate that Gamma-field irradiation and accelerator-field irradiation are simultaneously performed at treatment fractions 1, 6, 11, 16, and 21.

Each fraction group may also be associated with a Beam Sequence field (BeamSequence), which indicates treatment fields associated with the fraction group. The BeamSequence field may include information such as a treatment-field number, a treatment-field dose unit, and a control point list. The treatment-field dose unit distinguishes a type of the treatment field. For example, when the treatment-field dose unit is MINUTES, the treatment field is a Gamma field; when the treatment-field dose unit is MU, the treatment field is an accelerator field.

FIG. 4 is a schematic diagram illustrating content of a fused treatment plan based on a DICOM standard format according to an embodiment of the present application. As shown in FIG. 4, the Fraction Group Sequence field (FractionGroupSequence) may include two FractionGroup fields.

A first FractionGroup field may include a Fraction Group Number (FractionGroupNumber), a Referenced Beam Sequence list (ReferencedBeamSequence:List), and a Private Tag (PrivateTag). The ReferencedBeamSequence:List may include a plurality of items, and each item may include a Referenced Beam Number (ReferencedBeamNumber). The value of the FractionGroupNumber is 1, indicating the first fraction group. The ReferencedBeamSequence:List may include three items: item 1, item 2, and item 3. The ReferencedBeamNumber included in item 1 has a value of 1, indicating a first associated treatment field numbered 1. The ReferencedBeamNumber included in item 2 has a value of 2, indicating a second associated treatment field numbered 2. The ReferencedBeamNumber included in item 3 has a value of 3, indicating a third associated treatment field numbered 3.

The ReferencedBeamNumber may further be associated with the Beam Sequence (BeamSequence). The BeamSequence may be associated with a plurality of treatment-field fields. Each treatment-field field may include a Beam Number (BeamNumber), a Primary Dosimeter Unit (PrimaryDosimeterUnit), and a Control Point Sequence list (ControlPointSequence:List). The value of the BeamNumber is 1, indicating the first treatment field. The value of the treatment-field field in the BeamSequence corresponds to the value of the BeamNumber, so that a unique treatment field may be determined. The value of the PrimaryDosimeterUnit is MINUTES, indicating that the treatment field is a Gamma field.

The value of the PrivateTag is 1, 2, 3, 4, 5, indicating that execution fractions for the first fraction group are the first to fifth fractions. When an N-th treatment fraction is to be delivered to the target object, the radiotherapy device determines whether N is included in this group of associated fraction numbers. If N is included, the treatment fields associated with the fraction group are executed; if N is not included, the treatment fields associated with the fraction group are not executed.

A second FractionGroup field has field types the same as those of the first FractionGroup field but different values. For example, the value of the FractionGroupNumber is 2, indicating the second fraction group. The ReferencedBeamSequence:List may include 20 items: item 1, item 2, ..., item 8, ..., item 20. The ReferencedBeamNumber included in item 1 has a value of 4, indicating a fourth associated treatment field numbered 4. The ReferencedBeamNumber included in item 2 has a value of 5, indicating a fifth associated treatment field numbered 5. By analogy, the ReferencedBeamNumber included in item 8 has a value of 11, indicating an eleventh associated treatment field numbered 11.

The associated treatment fields may further be associated with the BeamSequence. The BeamSequence may be associated with a plurality of treatment-field fields. Each treatment-field field may include a BeamNumber, a PrimaryDosimeterUnit, and a ControlPointSequence:List. The value of the BeamNumber is 4, indicating the fourth treatment field. The value of the PrimaryDosimeterUnit is MU, indicating that the treatment field is an accelerator field. The value of the PrivateTag is 1, 2, 3, 4, 5, ..., 20, indicating that execution fractions for the second fraction group are the first to twentieth fractions.

FIG. 4 provides a brief description of content of a fused treatment plan based on the DICOM standard format according to some embodiments of the present application. The fused treatment plan may further include other relevant fields.

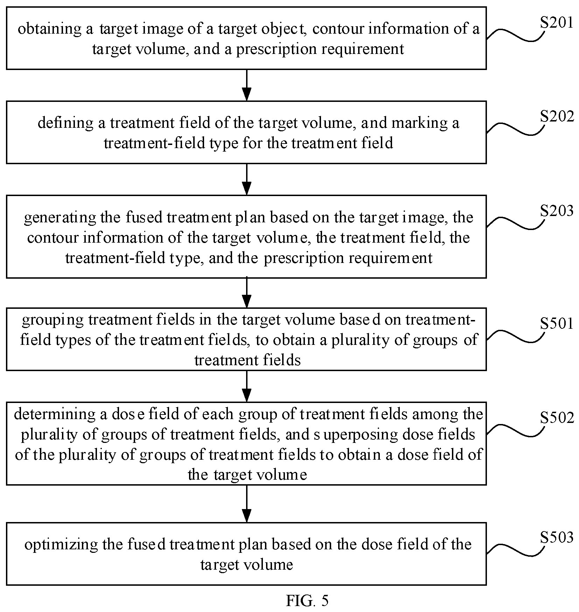

In some embodiments, after the fused treatment plan is obtained, the treatment planning device may further optimize the fused treatment plan. In this case, with reference to FIG. 2, as shown in FIG. 5, the treatment-plan generation method provided in some embodiments of the present application may further include the following steps.

S501, grouping treatment fields in the target volume based on treatment-field types of the treatment fields, to obtain a plurality of groups of treatment fields.

In some embodiments of the present application, the target volume may include multiple different types of treatment fields, and these treatment fields may undergo dose superposition in a same region. After dose superposition is performed, the dose field of the target volume may change. Therefore, some embodiments of the present application may further optimize the fused treatment plan based on the dose field of the target volume. In this case, the treatment planning device may first group the treatment fields in the target volume based on the treatment-field types of the treatment fields, to obtain the plurality of groups of treatment fields. Each group of treatment fields includes treatment fields of the same treatment-field type.

In combination with the foregoing example, when the fused treatment plan includes Gamma-field treatment-plan parameters and accelerator-field treatment-plan parameters, the plurality of groups of treatment fields may include a Gamma-field group and an accelerator-field group.

S502, determining a dose field of each group of treatment fields among the plurality of groups of treatment fields, and superposing dose fields of the plurality of groups of treatment fields to obtain a dose field of the target volume.

After the plurality of groups of treatment fields are obtained, the treatment planning device may calculate a dose field for each group of treatment fields. The treatment planning device may then superpose the dose fields of the plurality of groups of treatment fields to obtain the dose field of the target volume, for example, a dose field from which a dose-volume histogram (DVH) may be generated.

In this case, the method for superposing the dose fields of the plurality of groups of treatment fields to obtain the dose field of the target volume may specifically include the following.

The dose field of each group of treatment fields among the plurality of groups of treatment fields is partitioned according to a preset lattice size to obtain a dose lattice, also referred to herein as a dose grid, of each group of treatment fields, and dose lattice elements located at spatially corresponding positions in the dose lattices of the plurality of groups of treatment fields are superposed to obtain the dose field of the target volume.

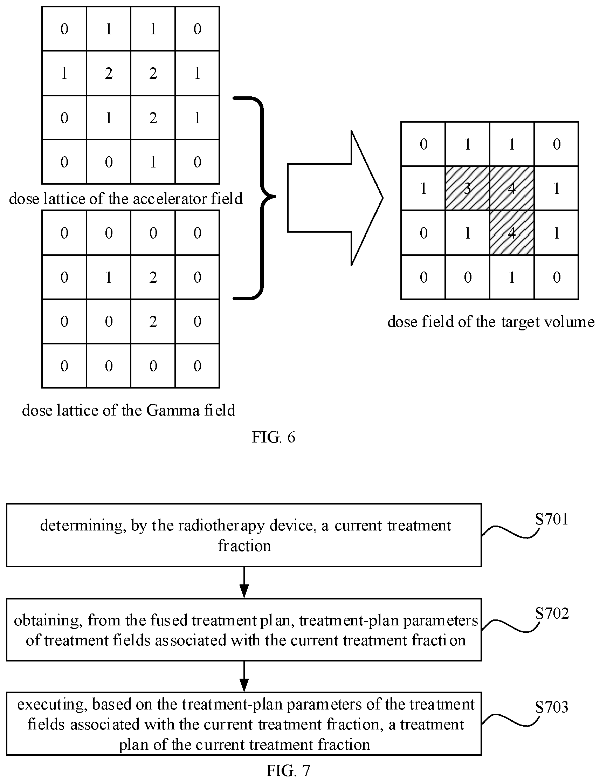

For example, as shown in FIG. 6, the target volume may include a dose lattice of a Gamma field and a dose lattice of an accelerator field. The dose lattice of the Gamma field and the dose lattice of the accelerator field are obtained by unifying, through algorithmic interpolation, an initial dose field of the Gamma field and an initial dose field of the accelerator field into dose lattices. The treatment planning device may superpose dose lattice elements located at corresponding positions in the dose lattice of the Gamma field and the dose lattice of the accelerator field to obtain the dose field of the target volume.

S503, optimizing the fused treatment plan based on the dose field of the target volume.

After the dose field of the target volume is obtained, the treatment planning device may optimize the fused treatment plan according to a difference between the dose field and the prescription requirement, for example, by adjusting treatment-plan parameters for treatment fields. Example implementations of the treatment-plan generation method are described above.

Example implementations of the treatment-plan execution method are described below with reference to FIG. 7.

FIG. 7 is a schematic flowchart of a treatment-plan execution method according to an embodiment of the present application. The treatment-plan execution method may be applied to the radiotherapy device 103 shown in FIG. 1. The treatment-plan execution method may include the following steps.

S701, determining, by the radiotherapy device, a current treatment fraction.

During execution of the generated fused treatment plan, the radiotherapy device may obtain a previous treatment record to determine the current treatment fraction, that is, the treatment fraction currently to be executed.

S702, obtaining, from the fused treatment plan, treatment-plan parameters of treatment fields associated with the current treatment fraction.

The fused treatment plan is generated based on the treatment-plan generation method described above. The fused treatment plan includes treatment-plan parameters for treatment fields of different treatment-field types. The treatment fields associated with the current treatment fraction include treatment fields of at least one treatment-field type.

In some examples, the radiotherapy device may parse the fused treatment plan and traverse a Fraction Group Sequence field (FractionGroupSequence) to obtain the fraction groups. The radiotherapy device may then obtain a private tag in each fraction group. The private tag marks a fraction to which the fraction group belongs. The radiotherapy device may determine whether the fraction group includes the current treatment fraction. If so, the radiotherapy device obtains, from the fraction group, treatment-plan parameters of the treatment fields associated with the current treatment fraction.

S703, executing, based on the treatment-plan parameters of the treatment fields associated with the current treatment fraction, a treatment plan of the current treatment fraction.

After obtaining the treatment-plan parameters of the treatment fields associated with the current treatment fraction, the radiotherapy device may merge treatment-plan parameters of treatment fields to be executed and sequentially execute the treatment-plan parameters of these treatment fields, thereby completing treatment.

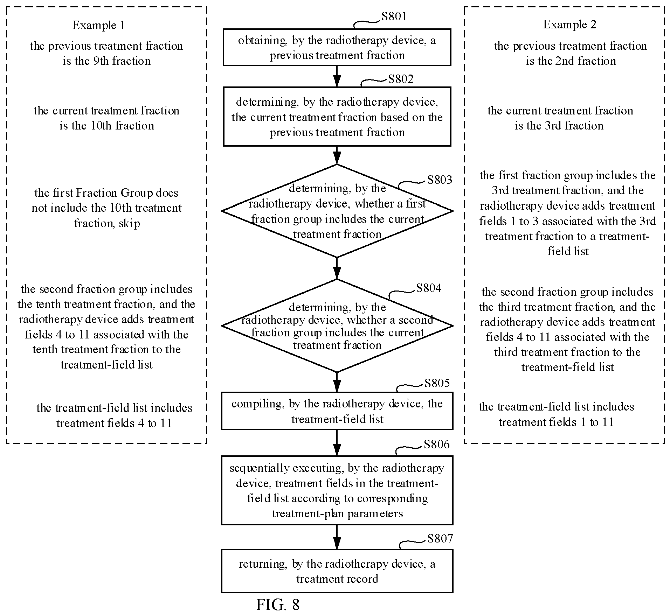

FIG. 8 is another schematic flowchart of a treatment-plan execution method according to an embodiment of the present application.

For example, as shown in FIG. 8, the treatment-plan execution method may include the following steps:

S801, obtaining, by the radiotherapy device, a previous treatment fraction.

For example, in Example 1, the previous treatment fraction is the ninth treatment fraction.

As another example, in Example 2, the previous treatment fraction is the second treatment fraction.

S802, determining, by the radiotherapy device, the current treatment fraction based on the previous treatment fraction.

For example, in Example 1, the previous treatment fraction is the ninth treatment fraction, and the radiotherapy device determines that the current treatment fraction is the tenth treatment fraction.

As another example, in Example 2, the previous treatment fraction is the second treatment fraction, and the radiotherapy device determines that the current treatment fraction is the third treatment fraction.

S803, determining, by the radiotherapy device, whether a first fraction group includes the current treatment fraction.

For example, in Example 1, the first fraction group does not include the tenth treatment fraction, and the radiotherapy device skips the first fraction group and proceeds to S804.

As another example, in Example 2, the first fraction group includes the third treatment fraction, and the radiotherapy device adds treatment fields 1 to 3 associated with the third treatment fraction to a treatment-field list.

S804, determining, by the radiotherapy device, whether a second fraction group includes the current treatment fraction.

For example, in Example 1, the second fraction group includes the tenth treatment fraction, and the radiotherapy device adds treatment fields 4 to 11 associated with the tenth treatment fraction to the treatment-field list.

As another example, in Example 2, the second fraction group includes the third treatment fraction, and the radiotherapy device adds treatment fields 4 to 11 associated with the third treatment fraction to the treatment-field list.

S805, compiling, by the radiotherapy device, the treatment-field list.

For example, in Example 1, the treatment-field list includes treatment fields 4 to 11.

As another example, in Example 2, the treatment-field list includes treatment fields 1 to 11.

S806, sequentially executing, by the radiotherapy device, treatment fields in the treatment-field list according to corresponding treatment-plan parameters.

S807, returning, by the radiotherapy device, a treatment record.

In some embodiments, the treatment-plan parameters include a treatment-head irradiation angle for the treatment field. When a plurality of treatment fields are associated with the current treatment fraction, the radiotherapy device may obtain, from the treatment-plan parameters of the treatment fields associated with the current treatment fraction, a treatment-head irradiation angle for each treatment field associated with the current treatment fraction.

The radiotherapy device may order, according to a preset order, the treatment-head irradiation angles for the treatment fields associated with the current treatment fraction, to obtain an ordering result. The radiotherapy device may execute the treatment plan of the current treatment fraction based on the treatment-plan parameters of the treatment fields associated with the current treatment fraction and the ordering result.

When the fused treatment plan is executed, the radiotherapy device may be a multi-head radiotherapy host. That is, the radiotherapy device may include a plurality of treatment heads, and different treatment heads may be configured to emit at least one of beams of different radiation types or beams of different irradiation types. For example, the radiotherapy device may include two treatment heads, one of which may be a Gamma Knife treatment head for rotational focusing radiotherapy, and the other of which may be an accelerator treatment head for conformal intensity-modulated radiotherapy. In a radiotherapy device including two treatment heads, the Gamma Knife treatment head and the accelerator treatment head have different included angles. For example, in a dual-head radiotherapy device of a manufacturer, the Gamma Knife treatment head and the accelerator treatment head form an included angle of 105 degrees in a clockwise direction. Assuming that the accelerator treatment head is used as gantry 0 degrees, the initial angle of the Gamma Knife treatment head is gantry 105 degrees. In this case, if the radiotherapy device appropriately orders different types of treatment fields, treatment-plan execution efficiency may be optimized and frequent reciprocating movement of the radiotherapy device may be avoided.

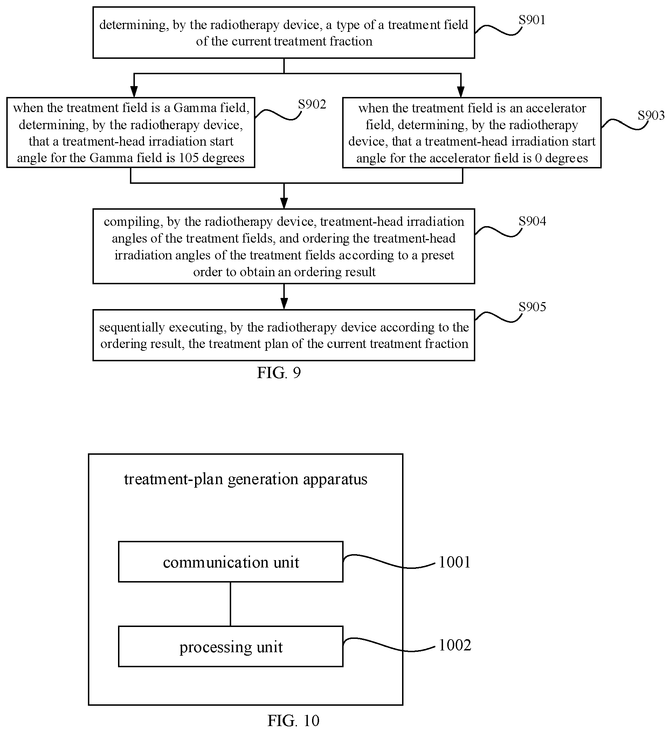

For example, as shown in FIG. 9, the treatment-plan execution method may include the following steps.

S901, determining, by the radiotherapy device, a type of a treatment field of the current treatment fraction.

S902, when the treatment field is a Gamma field, determining, by the radiotherapy device, that a treatment-head irradiation start angle for the Gamma field is 105 degrees.

S903, when the treatment field is an accelerator field, determining, by the radiotherapy device, that a treatment-head irradiation start angle for the accelerator field is 0 degrees.

S904, compiling, by the radiotherapy device, treatment-head irradiation angles of the treatment fields, and ordering the treatment-head irradiation angles of the treatment fields according to a preset order to obtain an ordering result.

S905, sequentially executing the treatment plan of the current treatment fraction according to the ordering result.

In some examples, the preset order may include a clockwise order or a counterclockwise order. The treatment-plan parameters of each treatment field may include a treatment-head irradiation angle for the treatment field. For example, for an accelerator field for single-point treatment, the treatment-plan parameters include a treatment-head irradiation angle for each treatment field. For a Gamma field for arc treatment, the treatment-plan parameters include a treatment-head irradiation start angle and a treatment-head irradiation end angle for the arc. The radiotherapy device may order treatment-head irradiation angles of the treatment fields based on information related to treatment-head irradiation angles in the treatment-plan parameters of each treatment field and based on the treatment-head irradiation start angle of each treatment field, to obtain the ordering result.

The radiotherapy device may execute the treatment plan starting from the treatment field having the smallest treatment-head irradiation angle according to the ordering result. Optionally, after execution of the treatment plan for the current treatment field is completed, the radiotherapy device may obtain a treatment-head irradiation angle at an end of treatment of the current treatment field. The radiotherapy device may then obtain two treatment-head irradiation angles adjacent to this treatment-head irradiation angle in the ordering result, namely a previous treatment-head irradiation angle and a next treatment-head irradiation angle. The radiotherapy device may determine differences between the two adjacent treatment-head irradiation angles and the treatment-head irradiation angle, and determine, as a next treatment field to be executed, a treatment field associated with the treatment-head irradiation angle having the smaller difference.

For example, when the treatment-head irradiation angle at the end of treatment of the current treatment field is 210 degrees, the radiotherapy device may obtain, from the ordering result, two treatment-head irradiation angles adjacent to this treatment-head irradiation angle, which are 180 degrees and 225 degrees. The radiotherapy device determines that the difference between 210 degrees and 225 degrees is smaller, and therefore determines the treatment field associated with the treatment-head irradiation angle of 225 degrees as the next treatment field to be executed. The radiotherapy device may perform the same operation for each subsequent treatment field until the treatment fields have been executed.

In some embodiments, when execution of the treatment plan of the current treatment fraction is interrupted, the radiotherapy device may determine a treatment field at which execution of the treatment plan of the current treatment fraction is interrupted.

In response to an instruction for resuming execution of the treatment plan of the current treatment fraction, the radiotherapy device may continue execution of the treatment plan of the current treatment fraction based on treatment-plan parameters for the treatment field at which execution of the current treatment fraction is interrupted.

For example, during execution of the current treatment fraction, if treatment is interrupted while the radiotherapy device is executing a specific treatment field, the radiotherapy device may record the specific treatment field as a treatment field at which execution is interrupted. When a resume-execution instruction is received, the radiotherapy device may continue execution based on treatment-plan parameters for the treatment field at which execution was interrupted, while avoiding unnecessary repeated execution of treatment fields that have already been completed.

During execution of the fused treatment plan, execution of the treatment plan of the current treatment fraction may be interrupted due to a power failure, a fault of the radiotherapy device, or another interruption event. In this case, the radiotherapy device may record the treatment field at which execution of the current treatment fraction is interrupted. After the radiotherapy device returns to normal, the radiotherapy device may resume execution based on treatment-plan parameters for the treatment field at which the current treatment fraction is interrupted in response to an instruction to resume execution of the treatment plan of the current treatment fraction, thereby completing execution of the treatment plan.

The example method implementations are described above. To implement the functions described above, the treatment planning device and the radiotherapy device may include at least one of hardware structures or software modules for the respective functions. Those skilled in the art should appreciate that the units and algorithm steps of the examples described in connection with the embodiments disclosed herein may be implemented in hardware or in a combination of hardware and computer software. Whether a function is performed by hardware or by computer software executed by hardware depends on the particular application and design constraints of the technical solution. Those skilled in the art may use different methods to implement the described functions for each particular application, and such implementation should not be considered as departing from the scope of embodiments of the present application.

Some embodiments of the present application may divide functional units of the treatment planning device and the radiotherapy device according to the method examples described above. For example, functional units may be divided according to respective functions, or two or more functions may be integrated into one processing unit. The integrated unit may be implemented in hardware or as a software functional unit. Unit division in some embodiments of the present application is illustrative and represents an example logical functional division, and other division manners may be used in actual implementation.

As shown in FIG. 10, some embodiments of the present application provide a treatment-plan generation apparatus. The apparatus is applicable to the treatment planning device described above and includes a communication unit 1001 and a processing unit 1002.

The communication unit 1001 is configured to obtain a target image of a target object, contour information of a target volume, and a prescription requirement.

The processing unit 1002 is configured to define a treatment field of the target volume and mark a treatment-field type of the treatment field. The treatment-field type indicates a treatment mode corresponding to the treatment field, and the treatment mode includes at least one of a treatment mode distinguished by radiation type or a treatment mode distinguished by irradiation type.

The processing unit 1002 is further configured to generate a fused treatment plan based on the target image, the contour information of the target volume, the treatment field, the treatment-field type, and the prescription requirement. The fused treatment plan includes treatment-plan parameters for treatment fields of different treatment-field types.

In some examples, the processing unit 1002 is further configured to group treatment fields in the target volume based on treatment-field types of the treatment fields, to obtain a plurality of groups of treatment fields.

The processing unit 1002 is further configured to determine a dose field of each group of treatment fields among the plurality of groups of treatment fields, and superpose dose fields of the plurality of groups of treatment fields to obtain a dose field of the target volume.

The processing unit 1002 is further configured to optimize the fused treatment plan based on the dose field of the target volume.

In some examples, the processing unit 1002 is specifically configured to perform the following.

The dose field of each group of treatment fields among the plurality of groups of treatment fields is partitioned according to a preset lattice size to obtain a dose lattice of each group of treatment fields.

Dose lattice elements located at corresponding positions in the dose lattices of the plurality of groups of treatment fields are superposed to obtain the dose field of the target volume.

In some examples, the prescription requirement includes prescription requirements for a plurality of target volumes of different types in the target volume.

In some examples, the prescription requirement is obtained in the following manner.

In response to a marking operation for a target-volume type of the target volume, the target-volume type of the target volume is obtained.

Based on the target-volume type of the target volume, prescription requirements are respectively set for target volumes of different target-volume types.

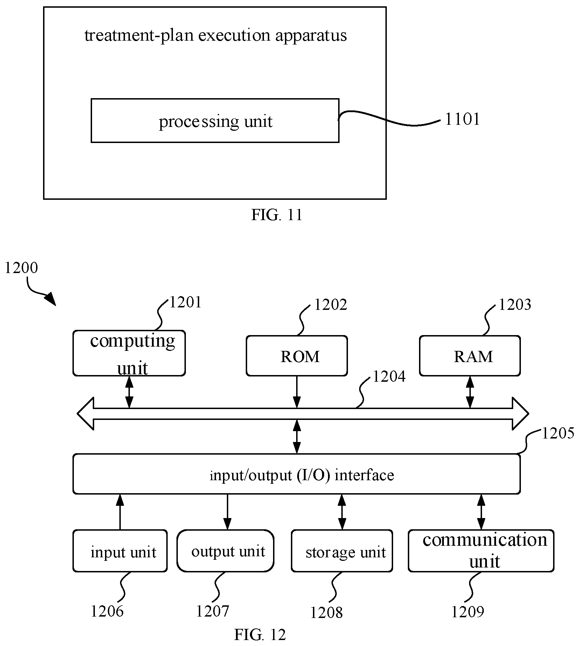

As shown in FIG. 11, some embodiments of the present application provide a treatment-plan execution apparatus. The apparatus is applicable to the radiotherapy device described above and includes a processing unit 1101.

The processing unit 1101 is configured to determine a current treatment fraction.

The processing unit 1101 is further configured to obtain, from a fused treatment plan, treatment-plan parameters for treatment fields associated with the current treatment fraction. The fused treatment plan is generated based on the treatment-plan generation method described above. The fused treatment plan includes treatment-plan parameters for treatment fields of different treatment-field types. The treatment fields associated with the current treatment fraction include treatment fields of at least one treatment-field type.

The processing unit 1101 is further configured to execute a treatment plan of the current treatment fraction based on the treatment-plan parameters of the treatment fields associated with the current treatment fraction.

In some examples, the treatment-plan parameters include a treatment-head irradiation angle for the treatment field. When a plurality of treatment fields are associated with the current treatment fraction, the processing unit 1101 is specifically configured to perform the following.

A treatment-head irradiation angle for each treatment field associated with the current treatment fraction is obtained from the treatment-plan parameters of the treatment fields associated with the current treatment fraction.

The treatment-head irradiation angles for the treatment fields associated with the current treatment fraction are ordered according to a preset order to obtain an ordering result.

The treatment plan of the current treatment fraction is executed based on the treatment-plan parameters of the treatment fields associated with the current treatment fraction and the ordering result.

In some examples, the processing unit 1101 is further configured to determine, when execution of the treatment plan of the current treatment fraction is interrupted, a treatment field at which execution of the current treatment fraction is interrupted.

The processing unit 1101 is further configured to continue execution of the treatment plan of the current treatment fraction, in response to an instruction for resuming execution of the treatment plan of the current treatment fraction, based on treatment-plan parameters for the treatment field at which the current treatment fraction is interrupted.

According to some embodiments of the present application, an electronic device is further provided. The electronic device includes at least one processor and a memory communicatively coupled to the at least one processor. The memory stores instructions executable by the at least one processor, and the instructions, when executed by the at least one processor, cause the at least one processor to perform the treatment-plan generation method or the treatment-plan execution method described herein.

According to some embodiments of the present application, a non-transitory computer-readable storage medium storing computer instructions is further provided. The computer instructions are configured to cause an electronic device to perform the treatment-plan generation method or the treatment-plan execution method described herein.

According to some embodiments of the present application, a computer program product is further provided. The computer program product includes a computer program that, when executed by a processor, implements the treatment-plan generation method or the treatment-plan execution method described herein.

FIG. 12 shows a schematic block diagram of an example electronic device 1200 that may be used to implement some embodiments of the present application. The electronic device is intended to represent various forms of digital computers, such as laptops, desktops, workstations, personal digital assistants, servers, blade servers, mainframes, and other suitable computers. The electronic device may also represent various forms of mobile devices, such as personal digital assistants, cellular phones, smart phones, wearable devices, and other similar computing devices. The components shown herein, their connections and relationships, and their functions are merely examples and are not intended to limit implementation of the present application described and/or claimed herein. In some embodiments, the electronic device may be the treatment planning device or the radiotherapy device shown in FIG. 1.

As shown in FIG. 12, the electronic device 1200 includes a computing unit 1201, which may perform various appropriate actions and processes according to a computer program stored in a read-only memory (ROM) 1202 or a computer program loaded from a storage unit 1208 into a random access memory (RAM) 1203. Various programs and data for operation of the electronic device 1200 may also be stored in the RAM 1203. The computing unit 1201, the ROM 1202, and the RAM 1203 are connected to one another through a bus 1204. An input/output (I/O) interface 1205 is also connected to the bus 1204.

A plurality of components in the electronic device 1200 are connected to the I/O interface 1205, including: an input unit 1206, such as a keyboard or a mouse; an output unit 1207, such as various types of displays or speakers; a storage unit 1208, such as a magnetic disk or an optical disk; and a communication unit 1209, such as a network card, a modem, or a wireless communication transceiver. The communication unit 1209 enables the electronic device 1200 to exchange information/data with other devices via a computer network, such as the Internet, and/or various telecommunication networks.

The computing unit 1201 may include various general-purpose and/or special-purpose processing components having processing and computing capabilities. Examples of the computing unit 1201 include, but are not limited to, a central processing unit (CPU), a graphics processing unit (GPU), dedicated artificial-intelligence (AI) computing chips, computing units running machine-learning model algorithms, a digital signal processor (DSP), and a suitable processor, controller, microcontroller, or the like. The computing unit 1201 performs the various methods and processes described above, such as the treatment-plan generation method or the treatment-plan execution method. For example, in some embodiments, the treatment-plan generation method or the treatment-plan execution method may be implemented as a computer software program tangibly embodied in a machine-readable medium, such as the storage unit 1208.

In some embodiments, a portion of the computer program may be loaded and/or installed onto the electronic device 1200 via the ROM 1202 and/or the communication unit 1209. When the computer program is loaded into the RAM 1203 and executed by the computing unit 1201, one or more steps of the treatment-plan generation method or the treatment-plan execution method described above may be performed. Alternatively, in some other embodiments, the computing unit 1201 may be configured to perform the treatment-plan generation method or the treatment-plan execution method by another suitable means, such as firmware.

Various implementations of the systems and techniques described herein may be implemented in digital electronic circuitry, integrated circuitry, field-programmable gate arrays (FPGAs), application-specific integrated circuits (ASICs), application-specific standard parts (ASSPs), systems on a chip (SOCs), complex programmable logic devices (CPLDs), computer hardware, firmware, software, and/or combinations thereof. These various implementations may include implementation in one or more computer programs that are executable and/or interpretable on a programmable system including at least one programmable processor. The programmable processor may be a special-purpose or general-purpose programmable processor configured to receive data and instructions from, and transmit data and instructions to, a storage system, at least one input device, and at least one output device.

Program code for implementing the methods of the present application may be written in a combination of one or more programming languages. Such program code may be provided to a processor or controller of a general-purpose computer, a special-purpose computer, or a programmable data processing apparatus, such that the program code, when executed by the processor or controller, causes the functions/operations specified in the flowcharts and/or block diagrams to be implemented. The program code may execute on a machine, partly on a machine as a stand-alone software package, partly on a machine and partly on a remote machine, or on a remote machine or server.

In the context of the present application, a machine-readable medium may be a tangible medium that may contain or store a program for use by, or in connection with, an instruction execution system, apparatus, or device. The machine-readable medium may be a machine-readable signal medium or a machine-readable storage medium. The machine-readable medium may include, for example, an electronic, magnetic, optical, electromagnetic, infrared, or semiconductor system, apparatus, or device, or a suitable combination thereof. More specific examples of a machine-readable storage medium include an electrical connection based on one or more wires, a portable computer disk, a hard disk, a random access memory (RAM), a read-only memory (ROM), an erasable programmable read-only memory (EPROM or flash memory), an optical fiber, a portable compact disc read-only memory (CD-ROM), an optical storage device, a magnetic storage device, or a suitable combination thereof.

To provide interaction with a user, the systems and techniques described herein may be implemented on a computer having a display device for displaying information to the user, such as a cathode-ray tube (CRT) or liquid-crystal display (LCD) monitor, and a keyboard and a pointing device, such as a mouse or a trackball, by which the user may provide input to the computer. Other kinds of devices may also be used to provide interaction with the user. For example, feedback provided to the user may be a form of sensory feedback, such as visual feedback, auditory feedback, or tactile feedback, and input from the user may be received in a form, including acoustic input, speech input, or tactile input.

The systems and techniques described herein may be implemented in a computing system that includes a backend component, such as a data server, a middleware component, such as an application server, a frontend component, such as a user computer having a graphical user interface or a web browser through which the user may interact with an implementation of the systems and techniques described herein, or a combination of such backend, middleware, or frontend components. Components of the system may be interconnected by a form or medium of digital data communication, such as a communication network. Examples of communication networks include a local area network (LAN), a wide area network (WAN), and the Internet.

A computer system may include a client and a server. The client and the server are generally remote from each other and typically interact through a communication network. The client and the server may have a client-server relationship by virtue of computer programs running on the respective computers. The server may be a cloud server, a server of a distributed system, or a server integrated with a blockchain.

The various processes described above may be reordered, added, or deleted. For example, steps described in the present application may be performed in parallel, sequentially, or in a different order, provided that desired results of the embodiments described herein may be achieved. The order of the steps is not limited by embodiments of the present application.

The embodiments described above are not intended to limit the scope of the claims. Those skilled in the art should understand that various modifications, combinations, subcombinations, and substitutions may be made according to design requirements and other factors. Modifications, equivalents, and variations based on the disclosure herein are within the scope of the claims.

## Claims

1. A treatment-plan generation method, **characterized in that** the method comprises:
obtaining, by a treatment planning device, a target image of a target object, contour information of a target volume, and a prescription requirement;
defining, by the treatment planning device, a treatment field of the target volume, and marking a treatment-field type for the treatment field, wherein the treatment-field type indicates a treatment mode corresponding to the treatment field, and wherein the treatment mode comprises at least one of a treatment mode distinguished by radiation type or a treatment mode distinguished by irradiation type; and
generating, by the treatment planning device, a fused treatment plan based on the target image, the contour information of the target volume, the treatment field, the treatment-field type, and the prescription requirement, wherein the fused treatment plan comprises treatment-plan parameters for treatment fields of different treatment-field types.

2. The method according to claim 1, **characterized in that** the method further comprises:
grouping, by the treatment planning device, treatment fields in the target volume based on treatment-field types of the treatment fields, to obtain a plurality of groups of treatment fields;
determining, by the treatment planning device, a dose field of each group of treatment fields among the plurality of groups of treatment fields, and superposing dose fields of the plurality of groups of treatment fields to obtain a dose field of the target volume; and
optimizing, by the treatment planning device, the fused treatment plan based on the dose field of the target volume.

3. The method according to claim 2, **characterized in that** superposing the dose fields of the plurality of groups of treatment fields to obtain the dose field of the target volume comprises:
partitioning, by the treatment planning device according to a preset lattice size, the dose field of each group of treatment fields among the plurality of groups of treatment fields to obtain a dose lattice of each group of treatment fields; and
superposing, by the treatment planning device, dose lattice elements located at corresponding positions in the dose lattices of the plurality of groups of treatment fields to obtain the dose field of the target volume.

4. The method according to claim 1, **characterized in that** the prescription requirement comprises prescription requirements for a plurality of target volumes of different target-volume types in the target volume.

5. The method according to claim 4, **characterized in that** the prescription requirement is obtained by:
obtaining, by the treatment planning device in response to a marking operation for a target-volume type of the target volume, the target-volume type of the target volume; and
setting, by the treatment planning device based on the target-volume type of the target volume, prescription requirements respectively for target volumes of different target-volume types.

6. A radiotherapy system, **characterized in that** the radiotherapy system comprises:
a treatment planning device configured to generate a fused treatment plan by implementing the method according to any one of claims 1 to 5; and
a radiotherapy device configured to execute the fused treatment plan generated by the treatment planning device.

7. The radiotherapy system according to claim 6, **characterized in that** the radiotherapy device is configured to:
determine a current treatment fraction;
obtain, from the fused treatment plan, treatment-plan parameters of treatment fields associated with the current treatment fraction, wherein the fused treatment plan comprises treatment-plan parameters for treatment fields of different treatment-field types, and wherein the treatment fields associated with the current treatment fraction comprise treatment fields of at least one treatment-field type; and
execute a treatment plan of the current treatment fraction based on the treatment-plan parameters of the treatment fields associated with the current treatment fraction.

8. The radiotherapy system according to claim 7, **characterized in that** the treatment-plan parameters comprise a treatment-head irradiation angle for the treatment field, and wherein, when a plurality of treatment fields are associated with the current treatment fraction, the radiotherapy device is configured to:
obtain, from the treatment-plan parameters of the treatment fields associated with the current treatment fraction, a treatment-head irradiation angle for each treatment field associated with the current treatment fraction;
order, according to a preset order, the treatment-head irradiation angles for the treatment fields associated with the current treatment fraction to obtain an ordering result; and
execute, based on the treatment-plan parameters of the treatment fields associated with the current treatment fraction and the ordering result, the treatment plan of the current treatment fraction.

9. The radiotherapy system according to claim 7, **characterized in that** the radiotherapy device is configured to:
determine a treatment field at which execution of the treatment plan of the current treatment fraction is interrupted, when execution of the treatment plan of the current treatment fraction is interrupted; and
continue, in response to an instruction for resuming execution of the treatment plan of the current treatment fraction, execution of the treatment plan of the current treatment fraction based on treatment-plan parameters for the treatment field at which execution of the current treatment fraction is interrupted.

10. The radiotherapy system according to claim 6, **characterized in that** the radiotherapy device comprises a multi-head radiotherapy host, wherein the multi-head radiotherapy host comprises a plurality of treatment heads, and wherein the plurality of treatment heads are configured to emit at least one of beams of different radiation types or beams of different irradiation types.

11. The radiotherapy system according to claim 6, **characterized in that** the radiotherapy device comprises a single-head multi-mode radiotherapy host, wherein the single-head multi-mode radiotherapy host comprises one treatment head, and wherein the one treatment head is configured to emit beams of a plurality of different irradiation types.

12. The radiotherapy system according to claim 6, **characterized in that** the radiotherapy device comprises a radiotherapy host system comprising a plurality of different radiotherapy hosts, wherein the different radiotherapy hosts correspond to different treatment modes, and wherein the treatment modes comprise at least one of treatment modes distinguished by radiation type or treatment modes distinguished by irradiation type.

13. The radiotherapy system according to claim 6, **characterized in that** the treatment planning device comprises at least one processor and a memory storing instructions that, when executed by the at least one processor, cause the treatment planning device to generate the fused treatment plan by implementing the method according to any one of claims 1 to 5.

14. The radiotherapy system according to claim 6, **characterized in that** the radiotherapy device comprises at least one processor and a memory storing instructions that, when executed by the at least one processor, cause the radiotherapy device to execute the fused treatment plan generated by the treatment planning device.

15. The radiotherapy system according to claim 14, **characterized in that** the instructions, when executed by the at least one processor of the radiotherapy device, cause the radiotherapy device to:
determine a current treatment fraction;
obtain, from the fused treatment plan, treatment-plan parameters of treatment fields associated with the current treatment fraction; and
execute a treatment plan of the current treatment fraction based on the treatment-plan parameters of the treatment fields associated with the current treatment fraction.
